(19) **Europäisches Patentamt / European Patent Office / Office européen des brevets**

(11) **EP 2 893 878 A1**

(12) # EUROPEAN PATENT APPLICATION
published in accordance with Art. 153(4) EPC

(43) Date of publication:
**15.07.2015 Bulletin 2015/29**

(21) Application number: **13834655.6**

(22) Date of filing: **04.09.2013**

(51) Int Cl.:
*A61B 5/22* (2006.01)   *A61B 5/11* (2006.01)
*A61B 5/0245* (2006.01)

(86) International application number:
**PCT/JP2013/073809**

(87) International publication number:
**WO 2014/038594 (13.03.2014 Gazette 2014/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.09.2012   JP 2012195035**

(71) Applicants:
- **Seiko Epson Corporation**
  **Shinjuku-ku**
  **Tokyo 163-0811 (JP)**
- **Tochikubo, Osamu**
  **Yokohama-shi**
  **Kanagawa 240-0024 (JP)**

(72) Inventors:
- **TOCHIKUBO, Osamu**
  **Yokohama-shi**
  **Kanagawa 240-0024 (JP)**
- **FURUTA, Naoshi**
  **Suwa-shi**
  **Nagano 392-8502 (JP)**
- **MITANI, Hitoshi**
  **Yokohama-shi**
  **Kanagawa 224-0037 (JP)**

(74) Representative: **Hoffmann Eitle**
**Patent- und Rechtsanwälte PartmbB**
**Arabellastraße 30**
**81925 München (DE)**

(54) **BIOINFORMATION PROCESSING SYSTEM, WEARABLE DEVICE, SERVER SYSTEM, AND CONTROL METHOD AND PROGRAM FOR BIOINFORMATION PROCESSING SYSTEM**

(57)   A biological information processing system, a wearable device, a server system, a method for controlling a biological information processing system, a program, and the like make it possible to accurately calculate health condition information by utilizing basal heart rate information. The biological information processing system includes a basal heart rate information acquisition section (120) that acquires basal heart rate information that represents the heart rate in a deep sleep state, a heart rate information acquisition section (110) that acquires heart rate information, and a health condition information calculation section (130) that calculates health condition information that represents a health condition based on relative information about the basal heart rate information and the heart rate information.

FIG. 1

**Description**

TECHNICAL FIELD

The present invention relates to a biological information processing system, a wearable device, a server system, a method for controlling a biological information processing system, a program, and the like.

BACKGROUND ART

A device and a system have been used that acquire heart rate information about the user using a given device, and provide information about the health condition and the like of the user based on the acquired information. The heart rate information may be acquired based on sensor information acquired from a pulse sensor or a heart rate sensor, for example.

The heart rate information (e.g., heart rate) can be used directly as an index value that represents the health condition of the user, and information about the daily lifestyle of the user can be calculated by performing given calculations using the heart rate information. For example, Patent Document 1 discloses a method that calculates the caloric expenditure of the user based on the heart rate information, and presents the calculated caloric expenditure to the user. The method disclosed in Patent Document 1 is particularly characterized in that the caloric expenditure calculation process is changed corresponding to whether the user is resting or exercising.

RELATED-ART DOCUMENT

PATENT DOCUMENT

Patent Document 1: JP-A-2009-285498

SUMMARY OF THE INVENTION

TECHNICAL PROBLEM

In Patent Document 1, the oxygen consumption $VO_2$ per minute of the user is estimated based on the heart rate information acquired from a heart rate sensor or the like, and the caloric expenditure is calculated from the estimated oxygen consumption $VO_2$. The maximum oxygen consumption $VO_{2m}$ per minute, the maximum heart rate $HR_m$, and the heart rate $HR_r$ at rest are used as parameters when estimating the oxygen consumption $VO_2$ per minute. However, since it is difficult to measure the maximum oxygen consumption $VO_{2m}$ per minute and the maximum heart rate $HR_m$, statistical values (virtual values) are used as the maximum oxygen consumption $VO_{2m}$ per minute and the maximum heart rate $HR_m$. Specifically, since the method disclosed in Patent Document 1 does not take account of an individual variation, the reliability of the calculated caloric expenditure may be low. Moreover, the method disclosed in Patent Document 1 can be applied only when calculating the caloric expenditure during exercise, and cannot determine the total daily caloric expenditure.

The heart rate HR increases due to not only physical exercise, but also mental activity (i.e., brain activity when making a mental count). Therefore, the heart rate $HR_r$ at rest may change due to brain activity and the like. However, since the method disclosed in Patent Document 1 does not take account of a change in the heart rate $HR_r$ at rest, it is difficult to accurately calculate the caloric expenditure during daily activity.

A system that performs caloric expenditure calculations has been widely known (see Patent Document 1). The health condition (quality of life (QOL)) of the user is largely affected by sleep, activity (caloric expenditure), stress, life rhythm, and the like. However, a system has not been proposed that calculates a wide variety of health condition information (e.g., daily caloric expenditure, deep sleep time information, and stress information (particularly mental stress information)) using information during a period without body motion in order to acquire information about sleep and the like.

Several aspects of the invention may provide a biological information processing system, a wearable device, a server system, a method for controlling a biological information processing system, a program, and the like that make it possible to accurately calculate the health condition information by utilizing basal heart rate information.

SOLUTION TO PROBLEM

According to one aspect of the invention, there is provided a biological information processing system comprising:

a basal heart rate information acquisition section that acquires basal heart rate information that represents a heart rate in a deep sleep state;

a heart rate information acquisition section that acquires heart rate information; and

a health condition information calculation section that calculates relative information about the basal heart rate information and the heart rate information, and calculates health condition information that represents a health condition based on the relative information.

According to one aspect of the invention, the health condition information is calculated based on the relative information about the basal heart rate information and the heart rate information. Since the basal heart rate information can be calculated with high reproducibility, differing from the heart rate information at rest or the like that changes depending on the mental condition, it is possible to accurately calculate the health condition information, for example.

In the biological information processing system,
the health condition information calculation section may calculate caloric expenditure information as the health condition information based on the relative information about the basal heart rate information and the heart rate information.

This makes it possible to calculate the caloric expenditure as the health condition information.

The biological information processing system may further comprise:

a body motion information acquisition section that acquires body motion information,

the health condition information calculation section may calculate the caloric expenditure information based on a first coefficient and the relative information, when it has been determined based on the body motion information that a body motion state has occurred, and may calculate the caloric expenditure information based on a second coefficient that differs from the first coefficient, and the relative information, when it has been determined based on the body motion information that a resting state has occurred.

According to this configuration, since the coefficient can be appropriately switched corresponding to the difference in caloric expenditure with respect to the heart rate between the body motion state or the resting state, it is possible to accurately calculate the caloric expenditure, for example.

In the biological information processing system,
the health condition information calculation section may calculate difference information about the basal heart rate information and the heart rate information as the relative information, may calculate a product of the first coefficient or the second coefficient, the difference information, and reference caloric expenditure per beat, and may calculate a sum of the calculated product and caloric expenditure corresponding to basal metabolism as the caloric expenditure information.

This makes it possible to specifically and easily calculate the caloric expenditure using the difference information or the like.

In the biological information processing system,
the heart rate information acquisition section may acquire the heart rate information in a given body motion state, and the health condition information calculation section may calculate the first coefficient based on the heart rate information in the given body motion state, the basal heart rate information, the relative information, and caloric expenditure corresponding to basal metabolism.

This makes it possible to calculate the first coefficient from the measured value utilizing the given body motion state, for example.

In the biological information processing system,
the health condition information calculation section may calculate deep sleep time information as the health condition information based on the relative information about the basal heart rate information and the heart rate information.

This makes it possible to calculate the deep sleep time information as the health condition information.

In the biological information processing system,
the health condition information calculation section may calculate the deep sleep time information based on the relative information about the heart rate information and a value obtained by multiplying the basal heart rate information by a sleep coefficient.

This makes it possible to appropriately calculate the deep sleep time information using the sleep coefficient, for example.

In the biological information processing system,
the health condition information calculation section may calculate the deep sleep time information by calculating a cumulative time in which a value represented by the heart rate information is equal to or smaller than the value obtained by multiplying the basal heart rate information by the sleep coefficient.

This makes it possible to use the cumulative time in which the user was determined to be in the deep sleep state as the deep sleep time information, for example.

In the biological information processing system,
the health condition information calculation section may calculate stress information as the health condition information based on the relative information about the basal heart rate information and the heart rate information.

This makes it possible to calculate the stress information as the health condition information.

The biological information processing system may further comprise:

a body motion information acquisition section that acquires body motion information,
the health condition information calculation section may calculate physical stress information as the stress information based on the relative information about the heart rate information and a value obtained by multiplying the basal heart rate information by a stress coefficient, when it has been determined based on the body motion information that a body motion state has occurred, and may calculate mental stress information as the stress information based on the relative information about the heart rate information and the value obtained by multiplying the basal heart rate information by the stress coefficient, when it has been determined based on the body motion information that a resting state has occurred.

This makes it possible to calculate the physical stress information or the mental stress information as the stress information corresponding to the body motion information.

In the biological information processing system,
the health condition information calculation section may calculate the physical stress information by calculating a cumulative time in which a value represented by the heart rate information is equal to or larger than the value obtained by multiplying the basal heart rate information by the stress coefficient, when it has been determined based on the body motion information that the body motion state has occurred.

This makes it possible to use the cumulative time in which physical stress was suffered by the user as the physical stress information, for example.

In the biological information processing system,
the health condition information calculation section may calculate the mental stress information by calculating a cumulative time in which a value represented by the heart rate information is equal to or larger than the value obtained by multiplying the basal heart rate information by the stress coefficient, when it has been determined based on the body motion information that the resting state has occurred.

This makes it possible to use the cumulative time in which mental stress was suffered by the user as the mental stress information, for example.

In the biological information processing system,
the basal heart rate information acquisition section may acquire the basal heart rate information based on information measured by a heart rate sensor or a pulse sensor.

This makes it possible to calculate the basal heart rate information using the heart rate sensor or the pulse sensor.

According to another aspect of the invention, there is provided a biological information processing system comprising:

a heart rate information acquisition section that acquires heart rate information;
a body motion information acquisition section that acquires body motion information;
a health condition information calculation section that calculates deep sleep time information, caloric expenditure information, and stress information based on the heart rate information and the body motion information; and
a display control section that displays information that represents a temporal distribution or a frequency distribution of the calculated deep sleep time information, the calculated caloric expenditure information, and the calculated stress information on a display section.

According to another aspect of the invention, there is provided a wearable device comprising the above biological information processing system.

According to another aspect of the invention, there is provided a server system comprising the above biological information processing system.

According to another aspect of the invention, there is provided a method for controlling a biological information processing system comprising:

performing a basal heart rate information acquisition process that acquires basal heart rate information that represents a heart rate in a deep sleep state;
performing a heart rate information acquisition process that acquires heart rate information; and
performing a health condition information calculation process that calculates relative information about the basal heart rate information and the heart rate information, and calculates health condition information that represents a health condition based on the relative information.

According to another aspect of the invention, there is provided a method for controlling a biological information processing system comprising:

performing a heart rate information acquisition process that acquires heart rate information;
performing a body motion information acquisition process that acquires body motion information;
performing a health condition information calculation process that calculates deep sleep time information, caloric expenditure information, and stress information based on the heart rate information and the body motion information; and
displaying information that represents a temporal distribution or a frequency distribution of the calculated deep sleep time information, the calculated caloric expenditure information, and the calculated stress information on a display section.

According to another aspect of the invention, there is provided a program that causes a computer to function as:

a basal heart rate information acquisition section that acquires basal heart rate information that represents a heart rate in a deep sleep state;
a heart rate information acquisition section that acquires heart rate information; and
a health condition information calculation section that calculates relative information about the basal heart rate information and the heart rate information, and calculates health condition information that represents a health condition based on the relative information.

According to another aspect of the invention, there is provided a program that causes a computer to function as:

a heart rate information acquisition section that acquires heart rate information;
a body motion information acquisition section that acquires body motion information;
a health condition information calculation section that calculates deep sleep time information, caloric expenditure information, and stress information based on the heart rate information and the body motion information; and
a display control section that displays information that represents a temporal distribution or a frequency distribution of the calculated deep sleep time information, the calculated caloric expenditure information, and the calculated stress information on a display section.

BRIEF DESCRIPTION OF DRAWINGS

FIG. 1 illustrates a system configuration example of a biological information processing system.
FIG. 2 illustrates an example of a system that includes a biological information processing system.
FIG. 3 illustrates an example of a basal heart rate information calculation method based on the distribution of heart rate information during sleep.
FIGS. 4A and 4B are views illustrating a coefficient corresponding to a period with body motion or a period without body motion.
FIG. 5 illustrates the correlation between basal metabolism calculated by a related-art method and basal metabolism calculated by a method according to one embodiment of the invention.
FIG. 6 illustrates an example of a home screen displayed on a display section.
FIG. 7 illustrates an example of a coefficient setting screen displayed on a display section.
FIG. 8 illustrates an example of a heart rate trend screen displayed on a display section.
FIGS. 9A and 9B illustrate examples of a screen that presents health condition information in an intuitive way.
FIG. 10 illustrates an example of an analysis screen displayed on a display section.

DESCRIPTION OF EMBODIMENTS

Exemplary embodiments of the invention are described below. Note that the following exemplary embodiments do not in any way limit the scope of the invention laid out in the claims. Note also that all of the elements described below in connection with the following exemplary embodiments should not necessarily be taken as essential elements of the invention.

1. Method

A method used in connection with several exemplary embodiments of the invention is described below. A method has been known that measures the heart rate (heart rate information) (HR) using a heart rate sensor or the like, and calculates the caloric expenditure from the oxygen consumption ($VO_2$) per minute estimated based on the heart rate information (see Patent Document 1). When the caloric intake exceeds the caloric expenditure, it can be determined that the user may show exacerbation of metabolic syndrome, for example. Therefore, the caloric expenditure can be used as health condition information that represents the health condition of the user.

In Patent Document 1, the following expression (1) is used when estimating the oxygen consumption per minute from the heart rate information, for example.

$$\frac{(VO_2 - VO_{2r})}{(VO_{2m} - VO_{2r})} \times 100(\%) = \frac{(HR - HR_r)}{(HR_m - HR_r)} \times 100(\%) \quad \cdots \cdots \quad (1)$$

where, $VO_{2m}$ is the maximum oxygen consumption per minute, $VO_{2r}$ is the oxygen consumption per minute at rest, $HR_m$ is the maximum heart rate (heart rate information), and $HR_r$ is the heart rate (heart rate information) at rest. In Patent Document 1, the maximum oxygen consumption $VO_{2m}$ per minute, the oxygen consumption $VO_{2r}$ per minute at rest, the maximum heart rate $HR_m$, and the heart rate $HR_r$ at rest are calculated, and the oxygen consumption $VO_2$ per minute is calculated from the calculated values and the measured heart rate (heart rate information) HR. Since the oxygen consumption $VO_2$ per minute and the caloric expenditure have a given relationship, it is possible to calculate the caloric expenditure from the estimated oxygen consumption $VO_2$ per minute.

However, it is not realistic to allow the subject to perform high-load exercise that causes the oxygen consumption per minute to almost reach the oxygen consumption $VO_{2m}$ per minute. Therefore, the maximum oxygen consumption $VO_{2m}$ per minute cannot be calculated from the measured value, and a given statistical value (virtual value) is used as the maximum oxygen consumption $VO_{2m}$ per minute. Likewise, the maximum heart rate $HR_m$ cannot be calculated from the measured value, and a given statistical value is used as the maximum heart rate $HR_m$. Therefore, the maximum oxygen consumption $VO_{2m}$ per minute and the maximum heart rate $HR_m$ do not take account of an individual variation between users. Specifically, the expression (1) is effective for calculating the tendency of the caloric expenditure and the like of a group consisting of a certain number of people, but is not necessarily effective for calculating the caloric expenditure of each user.

The heart rate (heart rate information) $HR_r$ at rest is used when estimating the oxygen consumption $VO_2$ per minute based on the expression (1). However, a problem may occur when using the heart rate (heart rate information) $HR_r$. Human activity with energy expenditure may be classified into physical activity (exercise) and mental activity. An increase in the heart rate (heart rate information) and the caloric expenditure is also observed during mental activity. Specifically, even if the user is in a physically resting state, the heart rate (heart rate information) $HR_r$ differs between the case where mental activity is absent (e.g., sleep state) and the case where mental activity is present (e.g., complex thinking state (e.g., during calculations) or tension state).

Patent Document 1 does not take account of a change in heart rate (heart rate information) $HR_r$ from the viewpoint of mental activity. Therefore, the expression (1) is effective when the user performs exercise under relatively high load, for example, but the caloric expenditure cannot be accurately calculated using the expression (1) when the user does not perform exercise, for example. The expression (1) does not pose a serious problem when the caloric expenditure during exercise is calculated. For example, related-art methods aim to inform the user of the caloric expenditure during exercise (e.g., running), and do not attach importance to the measurement of the caloric expenditure at rest. However, the caloric expenditure at rest serves as an important index when determining the health condition of the user during daily activity. For example, when determining the degree of exacerbation of metabolic syndrome, it is necessary to compare the caloric intake and the caloric expenditure on a basis of a given period (e.g., successive 24 hours) including a resting state. It is also necessary to accurately calculate the caloric expenditure during mental activity and the like at rest when using various other types of health condition information. In such a case, it is not appropriate to use the method that utilizes the expression (1) that may result in low accuracy at rest.

In order to solve the above problems, several embodiments of the invention propose a method that calculates the caloric expenditure without using the maximum oxygen consumption $VO_{2m}$ per minute, the oxygen consumption $VO_{2r}$ per minute at rest, the maximum heart rate (heart rate information) $HR_m$, the heart rate (heart rate information) $HR_r$ at rest, and the like. Specifically, basal heart rate information ($HR_0$) that is the heart rate information when the user is in a deep sleep state is calculated, and the caloric expenditure and the like are calculated using the basal heart rate information. The basal heart rate information is calculated using the method described later. Since the basal heart rate information is the heart rate information in a deep sleep state, the basal heart rate information does not change due to mental activity, differing from the heart rate (heart rate information) HR$_r$ at rest. Therefore, it is possible to accurately calculate the caloric expenditure during exercise (during a period with body motion (body motion state)) and during rest (during a period without body motion (resting state)). The details of the caloric expenditure calculation method are described later.

It is also possible to calculate other types of health condition information by utilizing the basal heart rate information. Specifically, whether or not the user is (was) in a deep sleep state may be determined using the heart rate information and the basal heart rate information, and deep sleep time information that represents the time in which the user was in a deep sleep state may be calculated from the determination results. Alternatively, whether or not stress is (was) suffered by the user may be determined using the heart rate information and the basal heart rate information, and stress information that represents the time in which stress was suffered by the user may be calculated from the determination results, for example.

A configuration example of a biological information processing system will be described first, and the basal heart rate information calculation method will be described thereafter. Caloric expenditure information, deep sleep time information, and stress information will then be described as specific examples of the health condition information calculated from the basal heart rate information, and an example that presents the calculated health condition information to the user (i.e., displays the calculated health condition information on a display section) will be described thereafter.

2. System configuration example

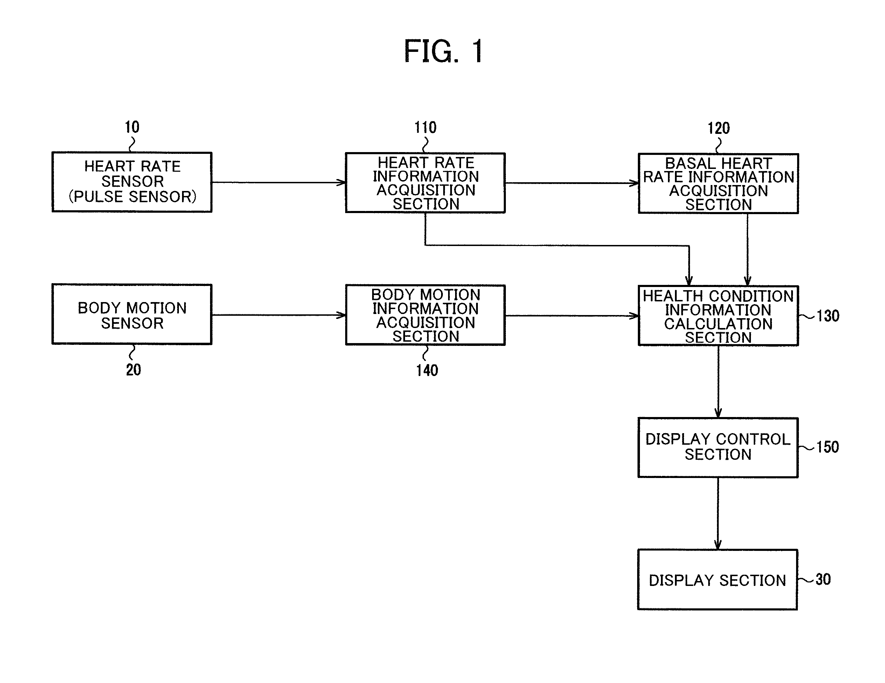

FIG. 1 illustrates a system configuration example of a biological information processing system according to one embodiment of the invention. The biological information processing system includes a heart rate sensor (or pulse sensor) 10, a body motion sensor 20, a heart rate information acquisition section 110, a basal heart rate information acquisition section 120, a health condition information calculation section 130, a body motion information acquisition section 140, a display control section 150, and a display section 30. Note that the biological information processing system is not limited to the configuration illustrated in FIG. 1. Various modifications may be made, such as omitting some of the elements illustrated in FIG. 1, or adding other elements.

The heart rate sensor (pulse sensor) 10 is connected to the heart rate information acquisition section 110, and the body motion sensor 20 is connected to the body motion information acquisition section 140. The heart rate information acquisition section 110 is connected to the basal heart rate information acquisition section 120 and the health condition information calculation section 130. The basal heart rate information acquisition section 120 and the body motion information acquisition section 140 are connected to the health condition information calculation section 130. The health condition information calculation section 130 is connected to the display control section 150, and the display control section 150 is connected to the display section 30.

A photoelectric sensor is used as the heart rate sensor (pulse sensor) 10, for example. In this case, light that has been applied to a living body, and reflected by the living body (or transmitted through the living body) may be detected using the photoelectric sensor, for example. Since the amount of absorption and the amount of reflection when light is applied to a living body differ corresponding to the blood flow within the vessels, sensor information detected by the photoelectric sensor include signals that correspond to the blood flow and the like, and information about pulsation can be acquired by analyzing the signals. Note that the heart rate sensor 10 is not limited to a photoelectric sensor. An electrocardiograph, an ultrasonic sensor, or the like may also be used as the heart rate sensor 10.

The body motion sensor 20 is a sensor that detects the body motion of the user. An acceleration sensor, an angular velocity sensor, or the like may be used as the body motion sensor 20. Note that another sensor may also be used as the body motion sensor 20.

The display section 30 displays a display screen that presents the calculated health condition information and the like. The display section 30 may be implemented by a liquid crystal display, an organic EL display, or the like.

The heart rate information acquisition section 110 acquires heart rate information based on the sensor information output from the heart rate sensor (pulse sensor) 10. The heart rate information acquisition section 110 acquires the heart rate information at a rate corresponding to the operation rate of the heart rate sensor 10, the calculation rate of the heart rate information acquisition section 110, and the like.

The basal heart rate information acquisition section 120 acquires basal heart rate information that represents the heart rate during deep sleep. The basal heart rate information acquisition method is described later. The basal heart rate information may be acquired based on the heart rate information output from the heart rate information acquisition section 110, or may be acquired based on the sensor information output from the heart rate sensor 10.

The health condition information calculation section 130 calculates health condition information that represents the health condition of the user. The health condition information is calculated based on the heart rate information, the basal heart rate information, body motion information, and the like. The details thereof are described later.

The body motion information acquisition section 140 acquires the body motion information based on the sensor information output from the body motion sensor. Since noise may occur due to the body motion of the user when the heart rate sensor 10 detects the sensor information, the body motion information may also be used when calculating the heart rate information in order to reduce noise in addition to the case of calculating the health condition information.

The display control section 150 displays the calculated health condition information on the display section 30. If the heart rate information and the like are presented directly to the user, it is difficult for the user (average user) who does not have medical knowledge and the like to determine his/her health condition. Therefore, it is desirable that the display control section 150 present the health condition of the user represented by the health condition information so that the user can easily determine his/her health condition. An example of the display screen is described later.

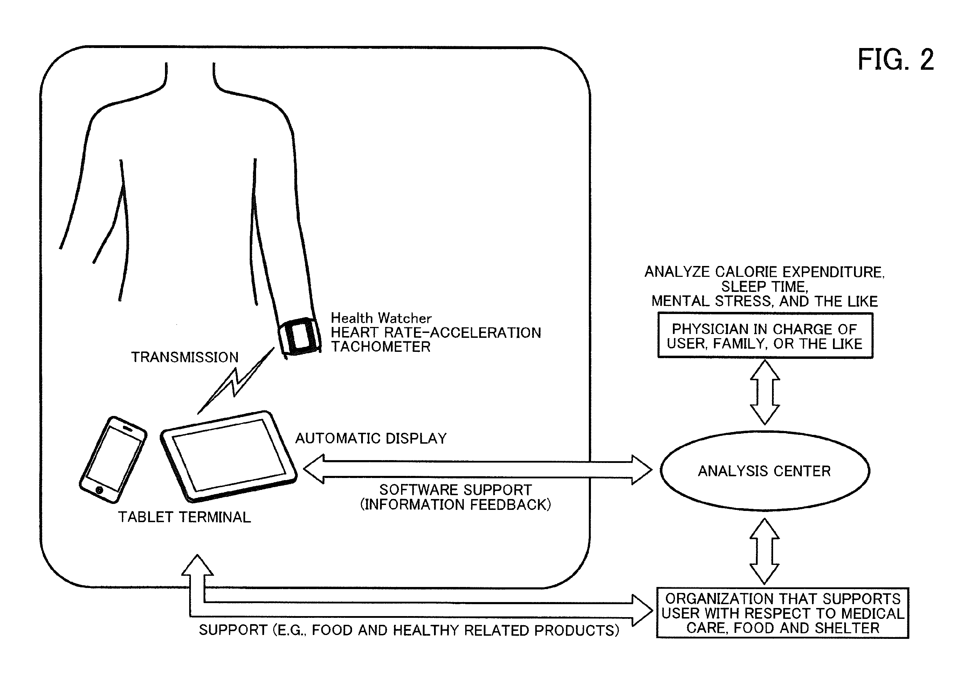

A typical usage example of the biological information processing system according to one embodiment of the invention is described below with reference to FIG. 2. The biological information processing system according to one embodiment of the invention does not necessarily acquire the biological information only during exercise. The biological information processing system according to one embodiment of the invention is designed to monitor the user as much as possible (i.e., monitor the user during exercise, at rest, during sleep, and the like). Therefore, since the heart rate sensor 10 and the body motion sensor 20 are worn by the user, it is necessary to use a low-invasive device that can be easily always worn by the user. For example, the watch-type wearable device (see "Health Watcher") illustrated in FIG. 2 may be used.

The watch-type wearable device normally includes the display section 30, and may include the heart rate information acquisition section 110, the basal heart rate information acquisition section 120, the health condition information calculation section 130, the body motion information acquisition section 140, and the display control section 150 in addition to the heart rate sensor 10 and the body motion sensor 20 (see FIG. 1). In this case, the biological information processing system is implemented by the watch-type wearable device.

However, since the display section of the watch-type wearable device has a small display area, it is difficult to display a number of pieces of health condition information at the same time. Therefore, the watch-type wearable device may be configured to display information that has been selected by the user from a plurality of pieces of information included in the health condition information. For example, when the user has selected the desired health condition information (i.e., health condition information that the user desires to check everyday) (e.g., stress information) on the watch-type wearable device, the selected health condition information may be displayed on the display section 30 of the watch-type wearable device. This makes it possible for the user to easily to check the desired health condition information.

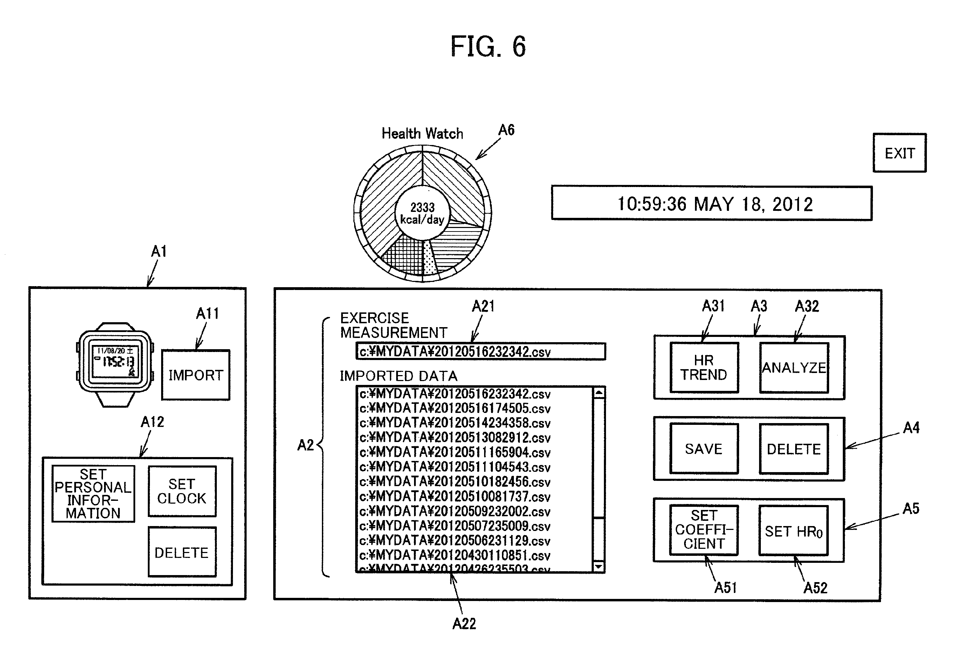

When displaying a display screen that presents a number of pieces of information at the same time (see FIGS. 6 to 10, for example), the visibility of the information and the like may be insufficient when the information is displayed on the display section of the watch-type wearable device having a small display area. Therefore, the display section of a tablet terminal or the like may be used as the display section 30 instead of the display section of the watch-type wearable device. In this case, the watch-type wearable device may implement the processes performed by the heart rate information acquisition section 110, the basal heart rate information acquisition section 120, the health condition information calculation section 130, the body motion information acquisition section 140, and the display control section 150, and the tablet terminal or the like may merely display the information. Alternatively, the watch-type wearable device may store and transmit the sensor information output from the heart rate sensor 10 and the body motion sensor 20, and the tablet terminal or the like may include the heart rate information acquisition section 110, the basal heart rate information acquisition section 120, the health condition information calculation section 130, the body motion information acquisition section 140, the display control section 150, and the display section 30.

The health condition information need not necessarily merely be observed by the user. For example, the health condition information may be transmitted to a health condition information analysis center (see FIG. 2) by utilizing the communication function of the tablet terminal or the like. The health condition information is transmitted to a server system provided in the analysis center, and stored therein. For example, when the physician in charge of the user, the user's family, or the like is allowed to observe the health condition information (e.g., access the server system), the physician can diagnose the health condition of the user, or the user's family can determine the health condition of the user, even if the user does not visit the physician or his/her family. When an organization that supports the user with respect to food, clothing and shelter is allowed to observe the health condition information, the organization can support the life of the user, for example.

When utilizing the server system, the server system may acquire the health condition information calculated by the health condition information calculation section 130 included in the watch-type wearable device, the tablet terminal, or the like. Alternatively, the server system may acquire the sensor information output from the heart rate sensor 10 and the body motion sensor 20, and calculate the health condition information through the heart rate information acquisition section 110, the basal heart rate information acquisition section 120, the health condition information calculation section 130, the body motion information acquisition section 140, and the like included in the server system. When the server system calculates the health condition information, it suffices that each user use a device that can merely transmit the sensor information, and receive the health condition information obtained by processing the sensor information (i.e., the device possessed by the user need not have high processing capacity).

3. Basal heart rate information

The basal heart rate information acquisition method is described below. The term "basal heart rate information" used herein refers to the heart rate information in a basal state. Specifically, the basal heart rate information is the heart rate information during deep sleep. The heart rate increases through physical activity and mental activity, and may change even at rest, as described above in connection with the heart rate $HR_r$ at rest. However, the heart rate information during deep sleep represents a minimum value (characteristic value) that changes to only a small extent as compared with a shallow sleep state and a waking state (with or without body motion), and the heart rate information about a single user during deep sleep shows a small day-to-day variation. Specifically, when the basal heart rate (basal heart rate information) is calculated from the heart rate information during deep sleep, the basal heart rate is based on the measured heart rate (heart rate information), and reflects an individual variation between the users. Moreover, the basal heart rate (basal heart rate information) that has been acquired (e.g., based on a single sleep period) can be continuously used for a long time.

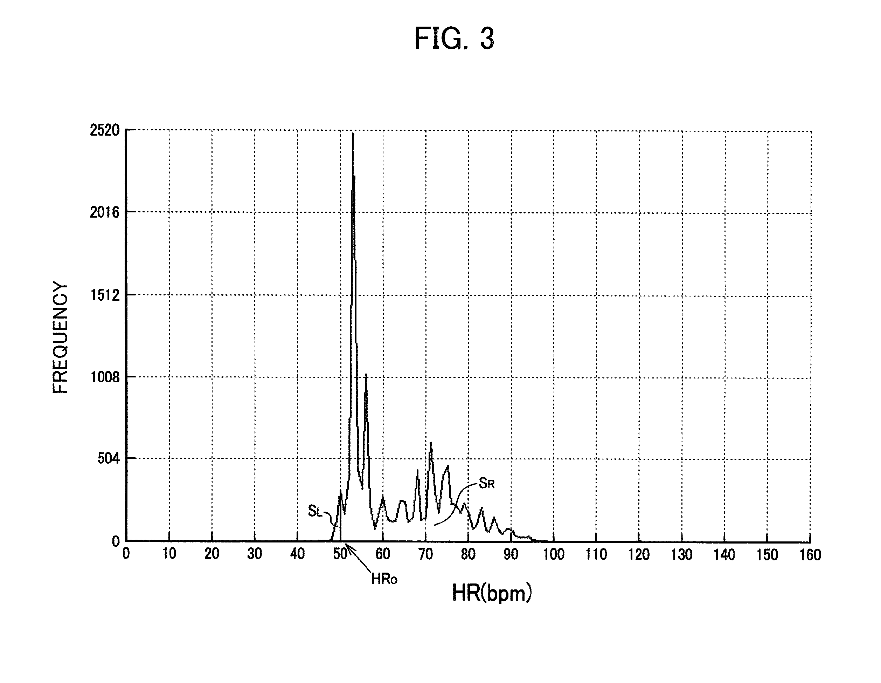

The basal heart rate information can be calculated using various methods. For example, the basal heart rate information may be calculated as illustrated in FIG. 3. FIG. 3 illustrates a graph of the heart rate (heart rate information) during sleep, wherein the horizontal axis indicates the heart rate (heart rate information), and the vertical axis indicates the number of times (frequency) each value appeared. It has been known that the heart rate (heart rate information) during sleep has a distribution (distribution having a zero point) similar to a gamma distribution. For example, the 1% lower-limit value in the heart rate frequency distribution in a period without body motion is determined to be the basal heart rate.

Specifically, the distribution is divided into a left region and a right region using a boundary line (i.e., a line parallel to the vertical axis), and the area of each region is calculated (see FIG. 3). A boundary point at which ratio of the area ($S_L$) of the left region to the area ($S_R$) of the right region is 1:99 (i.e., the area ($S_L$) of the left region accounts for 1% of the entire area) is determined. The heart rate (heart rate information) corresponding to the boundary point is determined to be the basal heart rate information $HR_0$.

The minimum heart rate (heart rate information) is not used as the basal heart rate information taking account of the effects of noise and the like. For example, when noise is superimposed on the sensor information or the like, the heart rate (heart rate information) may be very small. However, since it is not considered that the heart rate of a human becomes as low as 20 to 30 bpm taking account of the biological properties, a problem occurs if such a value is determined to be the basal heart rate. Therefore, the value calculated using the above method is used as the basal heart rate information instead of the minimum heart rate (heart rate information) in order to reduce the effects of noise and the like.

4. Calculation of caloric expenditure

4.1 Caloric expenditure calculation method

As described above, the method disclosed in Patent Document 1 estimates the oxygen consumption $VO_2$ from the heart rate (HR), the maximum oxygen consumption $VO_2$ ($VO_{2m}$), the maximum heart rate HR ($HR_m$), the oxygen consumption $VO_2$ ($VO_{2r}$) at rest, and the heart rate HR ($HR_r$) at rest based on the expression (1), and calculates the energy (caloric) expenditure EE per minute (EE=$VO_2 \times 5/1000$ kcal). However, the maximum oxygen consumption $VO_{2m}$, the maximum heart rate $HR_m$, the oxygen consumption $VO_{2r}$ at rest, and the heart rate $HR_r$ at rest do not sufficiently take account of an individual variation. Moreover, since the maximum oxygen consumption $VO_{2m}$ and the maximum heart rate $HR_m$ cannot be actually measured, and do not take account of the effects of action (ACT), the maximum oxygen consumption $VO_{2m}$ and the maximum heart rate $HR_m$ have low reliability. In particular, the method that utilizes the expression (1) poses a serious problem when monitoring the health condition of the user for a long time (e.g., whole day) irrespective of the presence or absence of body motion.

In order to solve this problem, the caloric expenditure is calculated based on the basal heart rate information $HR_0$ described above. Specifically, the expression (1) is transformed using the energy expenditure $EE_0$ per minute that corresponds to the basal heart rate information $HR_0$ to yield the following expression (2). In the expression (2), EE is the caloric expenditure per minute, $EE_0$ is the caloric expenditure EE in a basal state, and $EE_m$ is the maximum caloric expenditure EE.

$$\frac{(EE - EE_0)}{(EE_m - EE_0)} = \frac{(HR - HR_0)}{(HR_m - HR_0)} \quad \cdots \cdots \quad (2)$$

Solving the expression (2) with respect to the caloric expenditure EE yields the following expression (3).

$$EE = EE_0 + \frac{(EE_m / EE_0 - 1)}{(HR_m / HR_0 - 1)} \times \frac{EE_0}{HR_0} \times (HR - HR_0)$$

$$\text{If } \frac{(EE_m / EE_0 - 1)}{(HR_m / HR_0 - 1)} = x \quad , \quad HR - HR_0 = \Delta HR \quad , \text{ then}$$

$$EE = EE_0 + x \times \frac{EE_0}{HR_0} \times \Delta HR$$

$$\text{If } EE - EE_0 = \Delta EE \quad \text{then}$$

$$\Delta EE = x \times \frac{EE_0}{HR_0} \times \Delta HR \qquad \cdots \cdots (3)$$

The caloric expenditure $EE_0$ is a value that corresponds to the basal metabolism of the user per minute. Since the basal metabolism BM per day can be calculated using various methods, it is possible to calculate the caloric expenditure $EE_0$ in advance. The basal heart rate information $HR_0$ can be determined from the measured value (see above). The measured heart rate (heart rate information) may be used as the heart rate HR. Therefore, the caloric expenditure (caloric expenditure EE per minute) can be calculated by determining x in the expression (3).

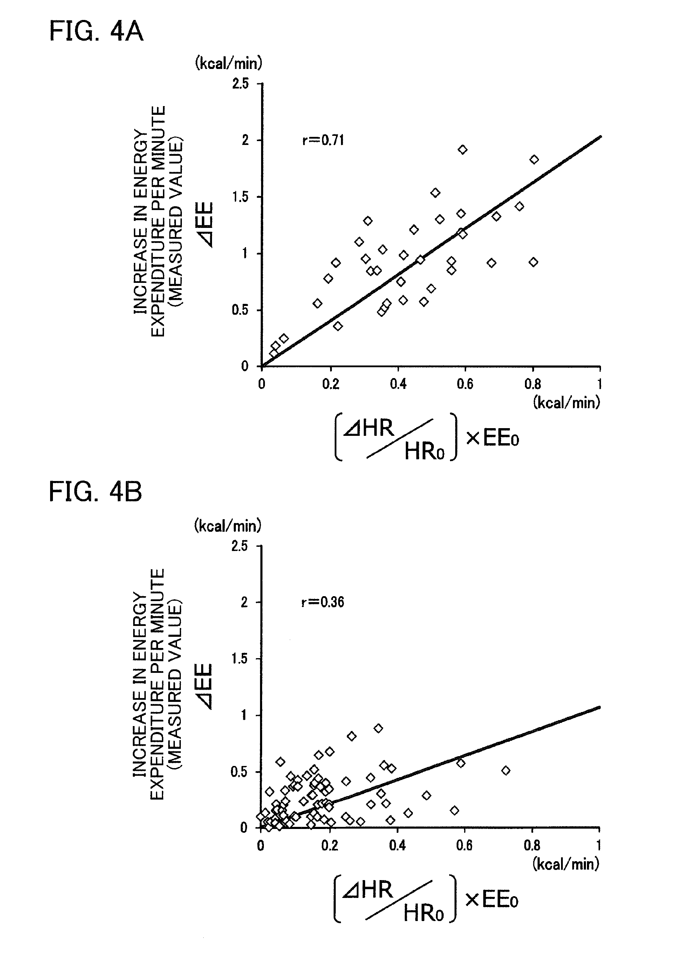

FIGS. 4A and 4B illustrate a graph in which the values are plotted wherein the vertical axis indicates the value $\Delta EE$ in the expression (3), and the horizontal axis indicates the value $(\Delta HR/HR_0) \times EE_0$ in the expression (3). FIG. 4A corresponds to a drawing in which the values when the user performed an action (during exercise or during a period with body motion) are plotted, and FIG 4B corresponds to a drawing in which the values when the user did not perform an action (at rest or during a period without body motion (e.g., a recumbent position, a sitting position, or a standing position at rest)) are plotted.

When the plotted points are approximated to a straight line, the slope of the straight line represents the coefficient x (see the plotted values and the expression (3)). As is clear from the comparison between FIG. 4A and FIG. 4B, the slope of the straight line illustrated in FIG. 4A that corresponds to the coefficient during a period with body motion is larger than the slope of the straight line illustrated in FIG. 4B that corresponds to the coefficient during a period without body motion. Specifically, the coefficient x during activity is larger than the coefficient x during non-activity, and it is necessary to change the coefficient corresponding to whether or not the user performed an action when calculating the energy expenditure (caloric expenditure).

In the expression (3), the value "$HR-HR_0$" is used as the index value $\Delta HR$ that represents the degree of change with respect to the reference value of the heart rate information. Specifically, the reference value is the basal heart rate information $HR_0$. However, it is likely that the user is in a standing position or a sitting position in a waking state regardless of whether or not the user makes a body motion. In this case, the heart rate is higher than the basal heart rate information $HR_0$ due to baroreceptor sensitivity even if both the physical activity and the mental activity are very weak. Therefore, it is desirable to use a value that takes account of an increase in heart rate as the reference value of the heart rate information instead of the basal heart rate information $HR_0$ when determining the value $\Delta HR$. In one embodiment of the invention, coefficients $\alpha$ and $\beta$ that are equal to or larger than 1 are set, "$\Delta HR=HR-\alpha HR_0$" is used during a period with body motion, and "$\Delta HR=HR-\beta HR_0$" is used during a period without body motion.

The embodiments of the invention utilize the following expressions (4) and (5) as the caloric expenditure calculation expression taking account of the fact that it is necessary to set the coefficient x to differ between a period with body motion and a period without body motion, and use the coefficients $\alpha$ and P (i.e., an increase in reference value) for the value $\Delta HR$ instead of the value "$HR-HR_0$". The expression (4) is an expression for calculating the caloric expenditure during a period with body motion, and the expression (5) is an expression for calculating the caloric expenditure during a period without body motion.

$$EE = EE_0 + x \times (HR - \alpha HR_0) \times \frac{EE_0}{HR_0} \quad \cdot \cdot \cdot \cdot \cdot \quad (4)$$

$$EE = EE_0 + y \times (HR - \beta HR_0) \times \frac{EE_0}{HR_0} \quad \cdot \cdot \cdot \cdot \cdot \quad (5)$$

As described above with reference to FIGS. 4A and 4B, the coefficient x is used for a period with body motion, and the coefficient y is used for a period without body motion. The coefficient x is a correction coefficient for an increase in stroke volume (SV) (i.e., the volume of blood pumped out per beat) during a period with body motion (i.e., an increase in stroke volume (SV) mainly due to muscular motion), and the coefficient y is a correction coefficient for an increase in stroke volume (SV) during a period without body motion (i.e., an increase in stroke volume (SV) due to mental activity or a change in position). Specifically, since the caloric expenditure (EE) per minute is proportional to the cardiac output (CO) (EE∝CO=SV×HR), but an increase in stroke volume (SV) with respect to an increase in the heart rate HR differs between a period with body motion and a period without body motion, the coefficients x and y are respectively used for a period with body motion and a period without body motion.

The coefficient $\alpha$ corrects the heart rate at rest in a waking state with body motion. It was experimentally confirmed that the heart rate at rest is higher than the basal heart rate (basal heart rate information) $HR_0$ by a factor of about 1.2 when the user is in a standing position in a waking state. Therefore, the coefficient $\alpha$ is normally set to 1.2. The coefficient $\beta$ corrects the initial value prior to mental activity or a change in position during a period without body motion. Since it is considered that such a state is close to the resting state, the coefficient P is normally set to 1.0.

4.2 Caloric expenditure $EE_0$ per minute corresponding to basal heart rate information $HR_0$

In order to calculate the caloric expenditure using the expression (4) or (5), it is necessary to determine the caloric expenditure $EE_0$ per minute that corresponds to the basal heart rate information $HR_0$. The basal metabolism BM per minute calculated from the basal metabolism BM per day corresponds to the caloric expenditure $EE_0$ per minute. The basal metabolism BM may be calculated using the widely-known Harris-Benedict equation.

Note that the caloric expenditure $EE_0$ per minute may be calculated based on the cardiac output (CO) per minute (i.e., a product of the heart rate HR and the stroke volume (SV)). Oxygen is bonded to hemoglobin in the lungs, transported by the heart, and released and utilized in tissue (brain and muscle) (the remainder is excreted from the lungs). The oxygen consumption $VO_2$ per minute and the cardiac output (CO) per minute have a proportional relationship ($VO_2 \propto CO = HR \times SV \propto EE$).

It was found that the heart rate becomes the basal heart rate $HR_0$ during slow-wave sleep (during deep sleep), and the cardiac index (CI) (CI=CO/BSA (BSA: body surface area)) during slow-wave sleep shows a small individual variation. The following expression (6) for calculating the cardiac output ($CO_0$) during sleep as a value that shows small individual variation was created by applying the above principle.

$$CO_0 = 6.9 \times Age^{-0.25} \times BSA \quad\quad\quad (6)$$

The oxygen consumption can be estimated from the expression (6), a typical blood hemoglobin concentration (male: 15 g/dl, female: 13.5 g/dl), the difference between the arterial oxygen saturation (97.5%) and the venous oxygen saturation (75%), and the amount (1.34 ml) of oxygen that is bonded to 1 g of hemoglobin. The basal metabolism BM can be estimated from the oxygen consumption corresponding to the cardiac output ($CO_0$) in the same manner as in the case of estimating the caloric expenditure EE from the oxygen consumption $VO_2$ per minute.

Specifically, the estimated basal metabolism BM is calculated by the following expression (7) (Hb: blood hemoglobin concentration) using the above values and a value for unit conversion. The estimated male basal metabolism $BM_m$ and the estimated female basal metabolism $BM_f$ are calculated by the following expression (8) using a specific value as the blood hemoglobin concentration Hb.

$$BM = CO_0 \times Hb \times 1.34 \times (0.975 - 0.75) \times 10 \times 60 \times 24 \times 5/1000 \qquad (7)$$

$$BM_m = 325.6 \times CO_0$$

$$BM_f = 293.0 \times CO_0 \qquad\qquad (8)$$

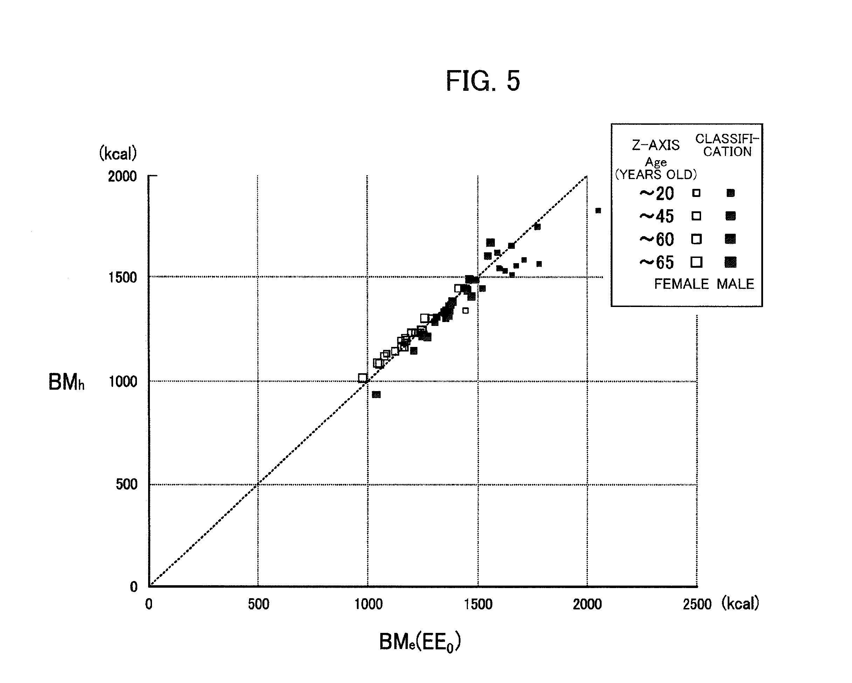

FIG. 5 illustrates a graph of the basal metabolism and the estimated basal metabolism of subjects who differ in age and sex, wherein the vertical axis indicates the basal metabolism calculated using the Harris-Benedict equation, and the horizontal axis indicates the estimated basal metabolism calculated using the expressions (6) and (8). In this case, the correlation coefficient r was 0.96 (i.e., a very high correlation was observed). Specifically, it is possible to accurately calculate the basal metabolism (and the caloric expenditure $EE_0$ per minute) using the method that utilizes the expressions (6) and (8).

4.3 Method for determining coefficients x and y

The coefficient x in the expression (4) and the coefficient y in the expression (5) are determined using the following method. The coefficients x and y may be determined using a given standard value, or may be calculated from a value measured after exercise under a given load.

The method that utilizes the standard value is described below. The coefficient x is close to the value "$(EE_m/EE_0-1)/(HR_m/HR_0-1)$" (see the expression (3)). It has been statistically (age: 20 to 70) known that the male maximum caloric expenditure $EE_m$ is 49-0.29×age, the female maximum caloric expenditure $EE_m$ is 49-0.29×age, and the maximum heart rate $HR_m$ is 220-age. The caloric expenditure $EE_0$ can be calculated from the male basal metabolism $BM_m$ or the female basal metabolism $BM_f$, and the heart rate HR can be acquired using the measured value output from the heart rate sensor or the like.

The coefficient x calculated using these values had an average value (about 5) close to $4.8 \pm 1.5$ (standard deviation (SD)). Therefore, "5" is used as the standard value of the coefficient x when the coefficient x is unknown. Note that a value statistically calculated by measuring the heart rate HR and the oxygen consumption $VO_2$ per minute during mental activity is used as the coefficient y. In one embodiment of the invention, "1.5" is used as the standard value of the coefficient y.

Note that the above method that determines the coefficient x calculates a rough estimate value of the coefficient x using statistical values as the maximum caloric expenditure $EE_m$ and the maximum heart rate HRm that cannot be easily measured. Therefore, it is difficult to deal with an individual variation between the users in the same manner as the method disclosed in Patent Document 1 (see the expression (1)).

The above problem can be solved by calculating the coefficient x from the measured value. Specifically, the expression (4) is transformed to yield the following expression (9).

$$x = (EE - EE_0) \times \frac{1}{(HR - \alpha HR_0) \times \dfrac{EE_0}{HR_0}} \quad \cdots\cdots (9)$$

The basal heart rate information $HR_0$ and the caloric expenditure $EE_0$ per minute can be calculated using the above methods, and it was experimentally found that "1.2" may preferably be used as the coefficient $\alpha$. The heart rate information HR can be calculated from the information output from the heart rate sensor or the like. Therefore, the coefficient x can be determined from the measured value provided that the caloric expenditure EE can be calculated. Since the method according to one embodiment of the invention aims to calculate the caloric expenditure EE, it is impossible to determine the caloric expenditure EE in advance if the target state is an arbitrary activity state (with or without body motion). However, when the target state is limited to a state in which the user performs exercise under given exercise load, it is possible to calculate the caloric expenditure EE during the exercise in advance. For example, when the user has performed 3-minute stepping exercise (2 steps per second)) (about 3 Mets), the caloric expenditure EE per minute during the exercise satisfies the following expression (10).

$$EE = 3 \times 1.05 \times \text{body weight} / 60 \qquad (10)$$

Specifically, since all of the values $HR_0$, $EE_0$, $\alpha$, HR, and EE can be determined and acquired when it was possible to instruct the user to perform given exercise, the coefficient x can be calculated from the measured value using the expression (9).

5. Calculation of deep sleep time information

Lack of sleep (e.g., when the deep sleep time is 4 hours or less) significantly affects the autonomic nerves on the next day, and adversely affects health. Therefore, the sleep time is an important index value when evaluating lifestyle. In particular, the time of deep sleep (slow-wave sleep) is important as an index value that represents the sleep state. For example, health is adversely affected when the sleep time is long, but the deep sleep time is short, and a subjective symptom such as fatigue occurs.

Therefore, information about whether or not the user is in a deep sleep state (i.e., information about the deep sleep time within 24 hours in which the user is in a deep sleep state in a narrow sense) is also calculated as the health condition information.

The heart rate (heart rate information) HR is close to the basal heart rate (basal heart rate information) $HR_0$ in a deep sleep state (see above). Therefore, whether or not the user is in a deep sleep state may be determined by comparing the heart rate HR with the basal heart rate $HR_0$. However, since the heart rate HR varies even in a deep sleep state, the heart rate HR may become higher than the basal heart rate $HR_0$. Therefore, the heart rate HR is compared with the value "$HR_0 \times$(sleep coefficient)" instead of the basal heart rate $HR_0$ (i.e., some margin is provided). Specifically, it is determined that the user is in a deep sleep state when the following expression (11) is satisfied, and the cumulative time within 24 hours in which the expression (11) is satisfied is determined to be the deep sleep time. The sleep coefficient in the expression (11) differs corresponding to each user. For example, a statistically calculated value (e.g., 1.12) may be used.

$$HR \leq HR_0 \times \text{(sleep coefficient)} \qquad (11)$$

6. Calculation of stress information

Stress information that represents the stress suffered by the user may also be used as an index value that represents the health condition of the user. The stress information may include information about physical stress that occurs due to physical activity during a period with body motion, and information about mental stress that occurs due to mental activity during a period without body motion.

The degree of physical stress and mental stress is considerably reflected in the heart rate (heart rate information) HR. Since it has been known that an increase in heart rate occurs during a period without body motion mainly due to brain activity, the mental stress can be evaluated by calculating the cumulative time in which an increase in heart rate equal to or larger than a given value was observed during a period without body motion. A stress coefficient is provided as a standard. It is determined that considerable mental stress is suffered by the user when the heart rate HR satisfies the following expression (12), and the cumulative time in which the expression (12) is satisfied is used as a mental stress index value (mental stress information).

$$HR \geq HR0 \times \text{(stress coefficient)} \qquad (12)$$

Since the stress coefficient differs corresponding to each user, the stress coefficient may be input externally. When the stress coefficient is unknown, or when it is desired to reduce the burden imposed on the user due to operation, for example, a statistically calculated value (e.g., 1.8) may be used as the stress coefficient.

The physical stress represents the stress suffered by the user during a period with body motion. The physical stress can be calculated by mainly taking account of an increase in heart rate due to muscular activity. Specifically, the physical stress is determined using the expression (12) in the same manner as the mental stress. Note that the physical stress determination process differs from the mental stress determination process in that the stress suffered by the user during a period with body motion is determined.

The presence or absence of body motion may be determined using various methods. For example, the presence or absence of body motion may be determined based on the sensor information output from the body motion sensor.

When the body motion sensor is an acceleration sensor, it may be determined that the user makes a body motion when the acceleration detection value (sensor information) output from the acceleration sensor is large, and it may be determined that the user does not make a body motion when the acceleration detection value is small. Alternatively, the frequency characteristics of the acceleration detection value (corresponding to the pitch during walking or running, for example) may be calculated, and the presence or absence of body motion may be determined based on the calculated frequency characteristics of the acceleration detection value instead of the magnitude of the acceleration detection value. Specifically, it suffices that the body motion sensor merely allow determination of the presence or absence of body motion when calculating the stress information. An acceleration sensor or another sensor may be used as the body motion sensor. The presence or absence of body motion may be determined based on the sensor information using an arbitrary method.

The stress information thus calculated can be used as an index value based on which it is determined that the mental stress suffered by the user is low when the above cumulative time is short, and the physical stress suffered by the user is appropriate when the above cumulative time is moderate (i.e., when the above cumulative time is long to such an extent that exercise is insufficient, and is short to such an extent that overload does not occur).

7. Display control

The caloric expenditure, the deep sleep time, and the stress information can be acquired as the health condition information using the method according to the embodiments of the invention (see above). If the acquired health condition information (value) is merely displayed, it may be difficult for the user to determine his/her health condition. Therefore, the acquired health condition information is presented to the user using a graph or the like so that the user (or the physician in charge of the user, a health adviser, or the like) can easily determine the health condition of the user.

Specific examples of the display screen are described below with reference to FIGS. 6 to 10. Note that the configuration of the display screen is not limited thereto. Although an example is described below in which the screen illustrated in FIG. 6 and the like is displayed on the tablet terminal or the like illustrated in FIG. 2 in order to display a certain amount of information at the same time, the information presentation display screen may be displayed on the display section of the watch-type wearable device by modifying the display section of the watch-type wearable device, or simplifying the display screen, for example.

FIG 6 illustrates an example of a home screen that is displayed when the watch-type wearable device is connected to the tablet terminal or the like. Cover information (e.g., personal information input mode (e.g., age, sex, height, weight, and ID), data file management, communication input-output management, basal heart rate information ($HR_0$) setting, and initial coefficient setting) is displayed within the home screen. The details thereof are described below.

The area A1 illustrated in FIG. 6 is used to perform a setting process and the like on the watch-type wearable device. Specifically, when the import button A11 is pressed in a state in which the watch-type wearable device is connected to the tablet terminal, the information acquired by the watch-type wearable device is imported into the tablet terminal. The information that is imported into the tablet terminal may include only the information obtained by the calculation process performed based on the heart rate HR and the basal heart rate $HR_0$ (e.g., caloric expenditure, deep sleep time, and stress information), or may further include the sensor information acquired by the heart rate sensor 10 and the body motion sensor 20. Note that various modifications may be made of the information that is imported into the tablet terminal.

The area A12 is used to register the user information, and set the clock, for example. Note that detailed description of the functions included in the area A12 is omitted.

The area A2 displays the file name under which the information imported by pressing the import button A11 is stored. Specifically, the area A21 may display the latest data file, and the area A22 may display the data files that have been imported.

The area A3 includes buttons used to display a temporal change in heart rate information (HR trend) included in the acquired information (e.g., when all of the heart rates HR have been acquired), the results of analysis based on the HR trend, and the like. An example of the display screen displayed when these buttons are pressed is described later.

The area A4 include buttons used to save and delete the data file.

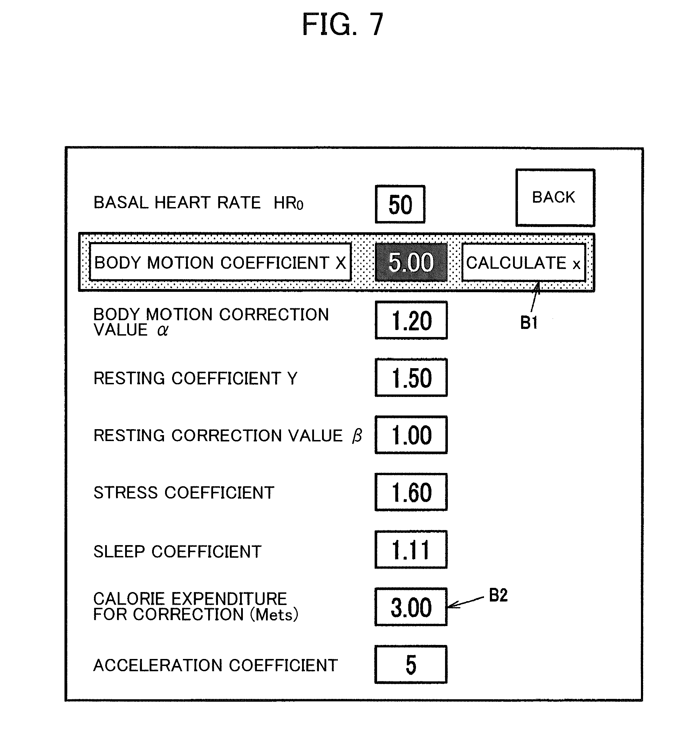

The area A5 is used to perform preparations for calculations of the health condition information. Specifically, the button A51 is used to display a screen for setting the coefficients used to calculate the health condition information, and the screen illustrated in FIG. 7 is displayed when the button A51 is pressed. The basal heart rate $HR_0$, the coefficients x, y, $\alpha$, and $\beta$ used to calculate the caloric expenditure, the sleep coefficient used to calculate the deep sleep time, the stress coefficient used to calculate the stress information, and the like can be set using the screen illustrated in FIG. 7. The coefficient x may be set based on the measured value (see above). In this case, the user presses the x calculation button B1, and starts a given exercise. It is possible to set the load during exercise (e.g., 3-minute stepping exercise (2 steps per second)) performed when setting the coefficient x based on the measured value (see "CORRECTED CALORIC EXPENDITURE (Mets)" (B2)). The acceleration coefficient is a threshold value relating to the acceleration detection value used when determining the presence or absence of body motion when an acceleration sensor is used as the body motion sensor. Note that the acceleration coefficient illustrated in FIG. 7 differs corresponding to the range of the acceleration sensor and the like. The unit for the acceleration coefficient is not necessarily "g" or "m/s$^2$" based on the standard gravitational acceleration.

The button A52 is used to set the basal heart rate $HR_0$. Since the basal heart rate $HR_0$ of a single user shows a small day-to-day variation (see above), the measured value can be continuously used. However, when the basal heart rate $HR_0$ has not been set based on the measured value, or when the user has instructed to reset the basal heart rate $HR_0$, for example, the basal heart rate $HR_0$ is set when the button A52 is pressed. Note that the basal heart rate $HR_0$ is set as described above with reference to FIG. 3.

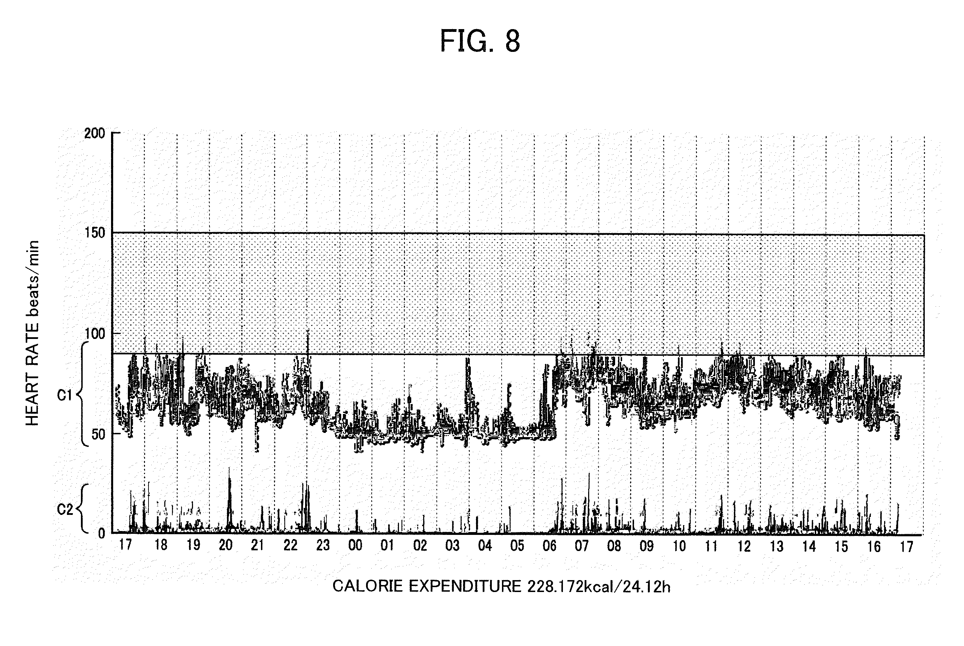

FIG. 8 illustrates an example of the screen displayed when the HR trend button A31 illustrated in FIG. 6 is pressed. FIG. 8 is a view illustrating a temporal change in heart rate (heart rate information) HR within a successive 24 hours, and a temporal change in caloric expenditure calculated based on the heart rate HR and the basal heart rate $HR_0$. The graph C1 illustrated in FIG. 8 shows a temporal change in heart rate HR, and the graph C2 illustrated in FIG. 8 shows a temporal change in caloric expenditure. The biological information (lifestyle information) about the user (e.g., the user was in a sleep state from about 0:00 a.m. to about 6:30 a.m.) can be obtained from FIG. 8.

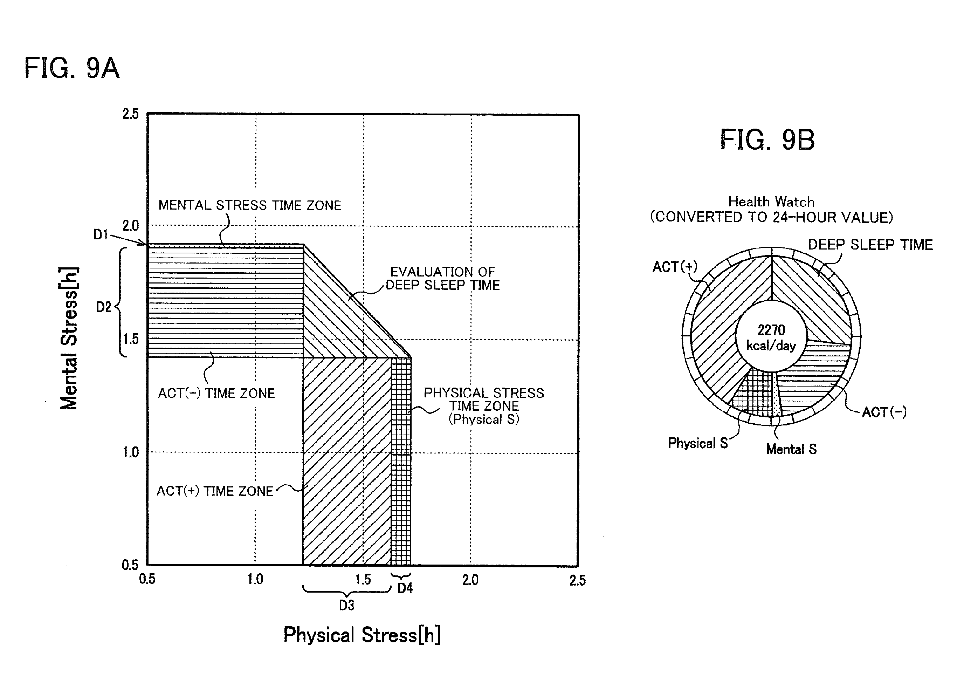

Since it is desirable to collectively present the health condition information so that the user can easily understand the health condition information, the display screen illustrated in FIG. 9A or 9B may be displayed in the area A6 illustrated in FIG. 6, for example. FIG. 6 illustrates an example in which the screen illustrated in FIG. 9B is displayed.

FIG 9B illustrates a graph that collectively shows the caloric expenditure, the deep sleep time, and the stress information during a day. In FIG. 9B, the deep sleep time refers to the time in which the user is in a deep sleep state, and ACT(-) refers to the time in which the user does not make a body motion, and mental stress is not suffered by the user. Mental S refers to the time in which the user does not make a body motion, and mental stress is suffered by the user. Physical S refers to the time in which the user makes a body motion, and physical stress is suffered by the user, and ACT(+) refers to the time in which the user makes a body motion, and physical stress is not suffered by the user. The 24-hour caloric expenditure is displayed at the center area in the circle graph.

It is possible to allow the user to intuitively determine the deep sleep time within 24 hours, the ratio of the time in which mental stress was suffered by the user, the ratio of the time in which physical stress was suffered by the user, and the like by displaying the graph illustrated in FIG. 9B. Specifically, since it is considered that healthy lifestyle satisfies conditions whereby rest (sleep) is sufficient, physical activity (physical stress) is moderate, mental stress is low, and calorie intake and caloric expenditure are well-balanced, it is possible to easily determine the health condition of the user by observing the graph illustrated in FIG. 9B from such a viewpoint.

Note that the user may not continuously wear the watch-type wearable device or the like for 24 hours taking account of charging the device and the like. In such a case, data acquired for a period of less than 24 hours is converted to 24-hour data in order to present the data (times) so that the user can easily determine the relative relationship between the data (times) (see FIG. 9B). For example, when the user wore the watch-type wearable device for 12 hours, each time is doubled. In this case, however, it may be difficult for the user to determine the actual time.

In order to avoid such a situation, the actual time may be displayed as illustrated in FIG. 9A without converting data to 24-hour data. In FIG. 9A, the vertical axis and the horizontal axis indicate time (unit: hours). D1 indicates the actual time in which mental stress was suffered by the user, D2 indicates the actual time in which the user did not make a body motion, and mental stress was suffered by the user, D3 indicates the actual time in which the user made a body motion, and physical stress was not suffered by the user, and D4 indicates the actual time in which physical stress was suffered by the user.

In FIG. 9A, the triangular area represents the deep sleep time. In this case, the deep sleep time may be represented by the color of the triangular area or the like instead of the size of the triangular area. For example, the triangular area may be displayed in green when the deep sleep time is sufficient (e.g., 7 hours or more), may be displayed in yellow when the deep sleep time is short to some extent (e.g., 4 to 7 hours), and may be displayed in red when the deep sleep time is insufficient (e.g., 4 hours or less).

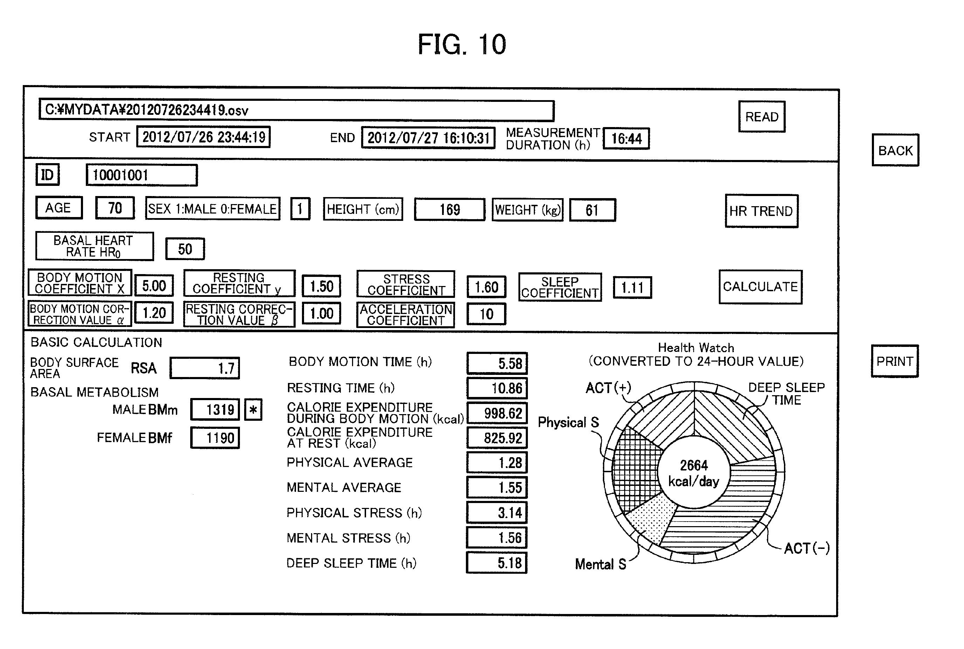

The graphical representation illustrated in FIGS. 9A and 9B and the like allows the user to intuitively determine his/her health condition, but makes it difficult for the user to know the accurate values. In order to deal with this problem, the analysis screen illustrated in FIG. 10 may be displayed when the analysis button A32 illustrated in FIG. 6 is pressed. The personal information about the user, the parameters used to calculate the health condition information, and the measured values (health condition information) are displayed within the analysis screen illustrated in FIG. 10, for example. The graph illustrated in FIG. 9B or the like may be displayed together with the analysis screen illustrated in FIG. 10. It is possible to allow the user to know the accurate values by displaying the analysis screen illustrated in FIG. 10, for example.

According to the embodiments of the invention, the biological information processing system includes the basal heart rate information acquisition section 120 that acquires the basal heart rate information that represents the heart rate in a deep sleep state, the heart rate information acquisition section 110 that acquires the heart rate information, and the health condition information calculation section 130 that calculates the relative information about the basal heart rate information and the heart rate information, and calculates the health condition information that represents the health condition based on the relative information (see FIG. 1).

The term "deep sleep state" used herein refers to a state in which the user is deep asleep (also referred to as "slow-wave sleep"). Specifically, when sleep is classified into rapid eye movement sleep (REM sleep) and non-rapid eye movement sleep (NREM sleep), and NREM sleep is classified into sleeping stages 1 to 4 in order from electroencephalographically shallow sleep (five sleeping stages in total), the deep sleep state corresponds to the sleeping stages 3 and 4.

The term "basal heart rate information" used herein refers to information that corresponds to the heart rate information in the deep sleep state. As described above with reference to FIG. 3, it was confirmed that the basal heart rate information represents a small value having high reproducibility (i.e., the minimum value or a value close to the minimum value when noise and the like are not taken into account) among the heart rate information acquired for a successive 24 hours.

The term "health condition information" used herein refers to information that is an index value that represents the health condition (degree of physical fitness) of the user for whom the heart rate information and the like are measured. The health condition information includes the caloric expenditure, the deep sleep time information, and the stress information.

The term "relative information about the basal heart rate information and the heart rate information" used herein refers to information that is determined by the relative relationship between the basal heart rate information and the heart rate information. Specifically, the relative information about the basal heart rate information and the heart rate information may be difference information including the difference between the basal heart rate information (or the value represented by the basal heart rate information) and the heart rate information (or the value represented by the heart rate information), or may be ratio information including the ratio of the heart rate information (or the value represented by the heart rate information) to the basal heart rate information (or the value represented by the basal heart rate information). The difference information and the ratio information are not limited to information that directly uses the basal heart rate information $HR_0$ and the heart rate information HR (i.e., $HR-HR_0$ and $HR/HR_0$). The difference information and the ratio information may be information determined by the difference or the ratio, such as information obtained by multiplying the difference or the ratio by a given coefficient, or information obtained by multiplying at least one of the heart rate information HR and the basal heart rate information $HR_0$ by a given coefficient, and calculating the difference between the heart rate information HR and the basal heart rate information $HR_0$.

The above configuration makes it possible to calculate the health condition information relating to lifestyle using the basal heart rate information. Since the basal heart rate information represents a value in a state in which mental activity is considered to be almost absent, it is unnecessary to take account of a change in the value represented by the basal heart rate information, differing from the heart rate information ($HR_r$) at rest that is used in the expression (1) and the like as the reference value for the heart rate information. Moreover, the basal heart rate information about a single user shows a small day-to-day variation. Therefore, it is possible to accurately calculate the health condition information by utilizing the basal heart rate information.

The health condition information calculation section 130 may calculate the caloric expenditure information as the health condition information based on the relative information about the basal heart rate information and the heart rate information.

This makes it possible to calculate the caloric expenditure information (i.e., caloric expenditure) as the health condition information. The caloric expenditure may be the caloric expenditure (EE) per unit time (e.g., during exercise), or may be the caloric expenditure per day. When calculating the caloric expenditure per day, it is useful to compare the caloric expenditure with the basal metabolism (BM) of the user in order to evaluate obesity, metabolic syndrome, and the like.

The biological information processing system may include the body motion information acquisition section 140 that acquires the body motion information (see FIG. 1). The health condition information calculation section 130 may calculate the caloric expenditure information based on a first coefficient and the relative information, when it has been determined based on the body motion information that a body motion state has occurred. The health condition information calculation section 130 may calculate the caloric expenditure information based on a second coefficient that differs from the first coefficient, and the relative information, when it has been determined based on the body motion information that a resting state has occurred.

This makes it possible to appropriately switch the coefficient used when calculating the caloric expenditure between a period with body motion and a period without body motion. This configuration is based on that fact that, when the plotted points are approximated to a straight line, the slope of the straight line (corresponding to the coefficient) differs to a large extent between a period with body motion and a period without body motion, as described above with reference to FIGS. 4A and 4B. When the stroke volume (SV) (i.e., the volume of blood pumped out per beat) is represented by $SV=\theta \times SV_0$ (where, $SV_0$ is the stroke volume (SV) in a basal state), an increase in the value $\theta$ (that relates to $x \times EE_0/HR_0$) is larger during physical activity than during mental activity. It may be considered that the above fact is due to this phenomenon.

The health condition information calculation section 130 may calculate the difference information about the basal heart rate information and the heart rate information as the relative information, calculate a product of the first coefficient (x) or the second coefficient (y), the difference information ($\Delta HR$), and the reference caloric expenditure ($EE_0/HR_0$) per beat, and calculate the sum of the calculated product and the caloric expenditure ($EE_0$) corresponding to basal metabolism as the caloric expenditure information.

This makes it possible to calculate the caloric expenditure using the expression (4) or (5). Note that the expressions (4) and (5) improve accuracy by utilizing the coefficient $\alpha$ or $\beta$ for the value $\Delta HR$ instead of using the value "$HR-HR_0$".

The heart rate information acquisition section 110 may acquire the heart rate information in a given body motion state, and the health condition information calculation section 130 may calculate the first coefficient based on the heart rate information in the given body motion state, the basal heart rate information, the relative information, and the caloric expenditure corresponding to basal metabolism.

This makes it possible to calculate the first coefficient x from the measured value based on the expression (9). The expression (3) calculates the caloric expenditure EE per minute provided that the first coefficient x is known. When the caloric expenditure EE per minute is known, the first coefficient x can be calculated by the expression (9) obtained by transforming the expression (3). In this case, it is necessary to prompt the user to perform a given exercise that allows estimation of the caloric expenditure EE per minute instead of arbitrary exercise.

The health condition information calculation section 130 may calculate the deep sleep time information as the health condition information based on the relative information about the basal heart rate information and the heart rate information.

Note that the deep sleep time information is not limited to the cumulative time (e.g., within 24 hours) in which the user was determined to be in the deep sleep state. For example, a daily change in time information that represents the time at which the user entered the deep sleep state may be used to determine the health condition. Time information that represents the time from the start of the resting state to the start of the deep sleep state, time information that represents the time from the start of sleep to the start of the deep sleep state, and the like are also useful for determining the health condition. The deep sleep time information may include such information.

This makes it possible to calculate the deep sleep time information as the health condition information. Since the basal heart rate information that is the heart rate information in the deep sleep state is used for the calculation process, it is possible to easily determine whether or not the user is in the deep sleep state by comparing the basal heart rate information and the heart rate information.

The health condition information calculation section 130 may calculate the deep sleep time information based on the relative information about the heart rate information and a value obtained by multiplying the basal heart rate information by the sleep coefficient.

This makes it possible to implement the determination process based on the relative information about the heart rate information and a value obtained by multiplying the basal heart rate information by the sleep coefficient. Since it was confirmed that the value represented by the heart rate information may change even in the deep sleep state, it is possible to implement an appropriate determination process by setting a moderate value that absorbs the change in the value represented by the heart rate information as the sleep coefficient.

The health condition information calculation section 130 may calculate the deep sleep time information by calculating the cumulative time in which the value represented by the heart rate information is equal to or smaller than a value obtained by multiplying the basal heart rate information by the sleep coefficient.

This makes it possible to calculate the cumulative time in which the user was in the deep sleep state as the deep sleep time information. For example, it is possible to determine whether or not the sleep time of the user is sufficient by calculating the cumulative time per day.

The health condition information calculation section 130 may calculate the stress information as the health condition information based on the relative information about the basal heart rate information and the heart rate information.

The term "stress information" used herein refers to information that represents whether or not stress (physical stress or mental stress) that can be distinguished from a normal state is suffered by the user. Since an increase in the value represented by the heart rate information is observed when physical stress or mental stress is suffered by the user, whether or not stress is suffered by the user can be determined based on a change in heart rate information. In particular, since the basal heart rate information that represents a small value having high reproducibility (i.e., the minimum value or a value close to the minimum value when noise and the like are not taken into account) is calculated, the relative information about the basal heart rate information and the heart rate information may be used.

This makes it possible to calculate the stress information as the health condition information. It is desirable that moderate physical stress be suffered by the user since exercise is insufficient when the physical stress suffered by the user is too low, and fatigue accumulates when the physical stress suffered by the user is too high. On the other hand, it is desirable that the mental stress suffered by the user be as low as possible.

The biological information processing system may include the body motion information acquisition section 140 that acquires the body motion information, and the health condition information calculation section 130 may calculate physical stress information as the stress information based on the relative information about the heart rate information and a value obtained by multiplying the basal heart rate information by the stress coefficient, when it has been determined based on the body motion information that the body motion state has occurred. The health condition information calculation section 130 may calculate mental stress information as the stress information based on the relative information about the heart rate information and a value obtained by multiplying the basal heart rate information by the stress coefficient, when it has been determined based on the body motion information that the resting state has occurred.

This makes it possible to determine whether the body motion state or the resting state has occurred based on the body motion information, and calculate the physical stress information (when the body motion state has occurred) or the mental stress information (when the resting state has occurred) using the expression (12).

The health condition information calculation section 130 may calculate the physical stress information by calculating the cumulative time in which the value represented by the heart rate information is equal to or larger than a value obtained by multiplying the basal heart rate information by the stress coefficient, when it has been determined based on the body motion information that the body motion state has occurred. The health condition information calculation section 130 may calculate the mental stress information by calculating the cumulative time in which the value represented by the heart rate information is equal to or larger than a value obtained by multiplying the basal heart rate information by the stress coefficient, when it has been determined based on the body motion information that the resting state has occurred.

This makes it possible to use the cumulative time in which it is determined that stress is suffered by the user, as the physical stress information and the mental stress information. It is desirable that the cumulative time used as the mental stress information be as short as possible. For example, when the cumulative period is set to a fixed value (e.g., 1 day), it is possible to set the upper limit or the like that is allowed taking account of health, and appropriately determine the health condition of the user. It is desirable that the cumulative time used as the physical stress information be a moderate (intermediate) value from the viewpoint of avoiding lack of exercise and an overloaded state. When the accumulation period is set to a fixed value (e.g., 1 day), it is possible to set the value range that is allowed taking account of health.

The basal heart rate information acquisition section 120 may acquire the basal heart rate information based on information measured by a heart rate sensor or a pulse sensor.

This makes it possible to calculate the basal heart rate information based on the information measured by the heart rate sensor (pulse sensor) 10. A specific basal heart rate information calculation method has been described above with reference to FIG. 3 and the like. Note that the basal heart rate information acquisition section 120 may acquire the sensor information directly from the heart rate sensor 10 or the like. However, when noise due to body motion is superimposed on the information measured by the heart rate sensor 10, a noise reduction process may be performed using the sensor information output from the heart rate sensor 10 and the sensor information output from the body motion sensor 20. In such a case, it is inefficient to cause the heart rate information acquisition section 110 and the basal heart rate information acquisition section 120 to independently perform the noise reduction process. Therefore, the basal heart rate information acquisition section 120 may calculate the basal heart rate information based on the output (heart rate information) from the heart rate information acquisition section 110 that has been subjected to the noise reduction process.

The biological information processing system may include the heart rate information acquisition section 110 that acquires the heart rate information, the body motion information acquisition section 140 that acquires the body motion information, the health condition information calculation section 130 that calculates the deep sleep time information, the caloric expenditure information, and the stress information based on the heart rate information and the body motion information, and the display control section 150 that displays information that represents the temporal distribution or the frequency distribution of the calculated deep sleep time information, the calculated caloric expenditure information, and the calculated stress information on the display section 30 (see FIG. 1).

This makes it possible to calculate the stress information that is not taken into account in the related-art method, and present the calculated health condition information to the user so that the user can intuitively and easily understand the health condition information (see FIGS. 9A and 9B).

The embodiments of the invention may also be applied to a wearable device that includes the biological information processing system, or a server system that includes the biological information processing system.

This makes it possible to implement the method according to the embodiments of the invention in various ways. The wearable device may be the watch-type wearable device illustrated in FIG. 2, for example. In this case, the biological information acquisition section (e.g., the heart rate sensor 10 and the body motion sensor 20 illustrated in FIG. 1) and the processing section (e.g., the health condition information calculation section 130) can be incorporated in a single device, and the wearable device can complete the process. The server system may be the server system that is provided in the analysis center illustrated in FIG. 2, for example. In this case, the biological information acquisition section is included in the wearable device, and the processing section is included in the server system. This makes it possible to distribute the processing load, and implement simplification in configuration and a reduction in cost of the wearable device, for example.

Note that part or most of the process performed by the biological information processing system and the like according to the embodiments of the invention may be implemented by a program. In this case, the biological information processing system and the like according to the embodiments of the invention are implemented by causing a processor (e.g., CPU) to execute a program. Specifically, a program stored in an information storage medium is read from the information storage medium, and a processor (e.g., CPU) executes the program read from the information storage medium. The information storage medium (computer-readable medium) stores a program, data, and the like. The function of the information storage medium may be implemented by an optical disk (e.g., DVD or CD), a hard disk drive (HDD), a memory (e.g., memory card or ROM), or the like. The processor (e.g., CPU) performs various processes according to the embodiments of the invention based on the program (data) stored in the information storage medium. Specifically, a program that causes a computer (i.e., a device that includes an operation section, a processing section, a storage section, and an output section) to function as each section according to the embodiments of the invention (i.e., a program that causes a computer to execute the process implemented by each section according to the embodiments of the invention) is stored in the information storage medium.

Although only some embodiments of the invention have been described in detail above, those skilled in the art will readily appreciate that many modifications are possible in the embodiments without materially departing from the novel teachings and advantages of the invention. Accordingly, all such modifications are intended to be included within scope of the invention. Any term cited with a different term having a broader meaning or the same meaning at least once in the specification and the drawings can be replaced by the different term in any place in the specification and the drawings. The configuration and the operation of the biological information processing system and the like are not limited to those described in connection with the above embodiments. Various modifications and variations may be made of the above embodiments as to the configuration and the operation of the biological information processing system and the like.

REFERENCE SIGNS LIST

10: heart rate sensor, 20: body motion sensor, 30: display section, 110: heart rate information acquisition section, 120: basal heart rate information acquisition section, 130: health condition information calculation section, 140: body motion information acquisition section, 150: display control section, BM: basal metabolism, CI: cardiac index, CO: cardiac output per minute, EE: caloric expenditure per minute, HR: heart rate information, $HR_0$: basal heart rate information, $VO_2$: oxygen consumption per minute

**Claims**

1. A biological information processing system comprising:

   a basal heart rate information acquisition section that acquires basal heart rate information that represents a heart rate in a deep sleep state;
   a heart rate information acquisition section that acquires heart rate information; and
   a health condition information calculation section that calculates relative information about the basal heart rate information and the heart rate information, and calculates health condition information that represents a health condition based on the relative information.

2. The biological information processing system as defined in claim 1,
   the health condition information calculation section calculating caloric expenditure information as the health condition information based on the relative information about the basal heart rate information and the heart rate information.

3. The biological information processing system as defined in claim 2, further comprising:

   a body motion information acquisition section that acquires body motion information,
   the health condition information calculation section calculating the caloric expenditure information based on a first coefficient and the relative information, when it has been determined based on the body motion information that a body motion state has occurred, and calculating the caloric expenditure information based on a second coefficient that differs from the first coefficient, and the relative information, when it has been determined based on the body motion information that a resting state has occurred.

**4.** The biological information processing system as defined in claim 3,
the health condition information calculation section calculating difference information about the basal heart rate information and the heart rate information as the relative information, calculating a product of the first coefficient or the second coefficient, the difference information, and reference caloric expenditure per beat, and calculating a sum of the calculated product and caloric expenditure corresponding to basal metabolism as the caloric expenditure information.

**5.** The biological information processing system as defined in claim 3 or 4,
the heart rate information acquisition section acquiring the heart rate information in a given body motion state, and the health condition information calculation section calculating the first coefficient based on the heart rate information in the given body motion state, the basal heart rate information, the relative information, and caloric expenditure corresponding to basal metabolism.

**6.** The biological information processing system as defined in claim 1,
the health condition information calculation section calculating deep sleep time information as the health condition information based on the relative information about the basal heart rate information and the heart rate information.

**7.** The biological information processing system as defined in claim 6,
the health condition information calculation section calculating the deep sleep time information based on the relative information about the heart rate information and a value obtained by multiplying the basal heart rate information by a sleep coefficient.

**8.** The biological information processing system as defined in claim 7,
the health condition information calculation section calculating the deep sleep time information by calculating a cumulative time in which a value represented by the heart rate information is equal to or smaller than the value obtained by multiplying the basal heart rate information by the sleep coefficient.

**9.** The biological information processing system as defined in claim 1,
the health condition information calculation section calculating stress information as the health condition information based on the relative information about the basal heart rate information and the heart rate information.

**10.** The biological information processing system as defined in claim 9, further comprising:

a body motion information acquisition section that acquires body motion information,
the health condition information calculation section calculating physical stress information as the stress information based on the relative information about the heart rate information and a value obtained by multiplying the basal heart rate information by a stress coefficient, when it has been determined based on the body motion information that a body motion state has occurred, and calculating mental stress information as the stress information based on the relative information about the heart rate information and the value obtained by multiplying the basal heart rate information by the stress coefficient, when it has been determined based on the body motion information that a resting state has occurred.

**11.** The biological information processing system as defined in claim 10,
the health condition information calculation section calculating the physical stress information by calculating a cumulative time in which a value represented by the heart rate information is equal to or larger than the value obtained by multiplying the basal heart rate information by the stress coefficient, when it has been determined based on the body motion information that the body motion state has occurred.

**12.** The biological information processing system as defined in claim 10 or 11,
the health condition information calculation section calculating the mental stress information by calculating a cumulative time in which a value represented by the heart rate information is equal to or larger than the value obtained by multiplying the basal heart rate information by the stress coefficient, when it has been determined based on the body motion information that the resting state has occurred.

**13.** The biological information processing system as defined in any one of claims 1 to 12,
the basal heart rate information acquisition section acquiring the basal heart rate information based on information measured by a heart rate sensor or a pulse sensor.

**14.** A biological information processing system comprising:

a heart rate information acquisition section that acquires heart rate information;
a body motion information acquisition section that acquires body motion information;
a health condition information calculation section that calculates deep sleep time information, caloric expenditure information, and stress information based on the heart rate information and the body motion information; and
a display control section that displays information that represents a temporal distribution or a frequency distribution of the calculated deep sleep time information, the calculated caloric expenditure information, and the calculated stress information on a display section.

**15.** A wearable device comprising the biological information processing system as defined in any one of claims 1 to 14.

**16.** A server system comprising the biological information processing system as defined in any one of claims 1 to 14.

**17.** A method for controlling a biological information processing system comprising:

performing a basal heart rate information acquisition process that acquires basal heart rate information that represents a heart rate in a deep sleep state;
performing a heart rate information acquisition process that acquires heart rate information; and
performing a health condition information calculation process that calculates relative information about the basal heart rate information and the heart rate information, and calculates health condition information that represents a health condition based on the relative information.

**18.** A method for controlling a biological information processing system comprising:

performing a heart rate information acquisition process that acquires heart rate information;
performing a body motion information acquisition process that acquires body motion information;
performing a health condition information calculation process that calculates deep sleep time information, caloric expenditure information, and stress information based on the heart rate information and the body motion information; and
displaying information that represents a temporal distribution or a frequency distribution of the calculated deep sleep time information, the calculated caloric expenditure information, and the calculated stress information on a display section.

**19.** A program that causes a computer to function as:

a basal heart rate information acquisition section that acquires basal heart rate information that represents a heart rate in a deep sleep state;
a heart rate information acquisition section that acquires heart rate information; and
a health condition information calculation section that calculates relative information about the basal heart rate information and the heart rate information, and calculates health condition information that represents a health condition based on the relative information.

**20.** A program that causes a computer to function as:

a heart rate information acquisition section that acquires heart rate information;
a body motion information acquisition section that acquires body motion information;
a health condition information calculation section that calculates deep sleep time information, caloric expenditure information, and stress information based on the heart rate information and the body motion information; and
a display control section that displays information that represents a temporal distribution or a frequency distribution of the calculated deep sleep time information, the calculated caloric expenditure information, and the calculated stress information on a display section.

# FIG. 1

HEART RATE SENSOR (PULSE SENSOR) — 10

HEART RATE INFORMATION ACQUISITION SECTION — 110

BASAL HEART RATE INFORMATION ACQUISITION SECTION — 120

BODY MOTION SENSOR — 20

BODY MOTION INFORMATION ACQUISITION SECTION — 140

HEALTH CONDITION INFORMATION CALCULATION SECTION — 130

DISPLAY CONTROL SECTION — 150

DISPLAY SECTION — 30

EP 2 893 878 A1

FIG. 2

Health Watcher
HEART RATE-ACCELERATION
TACHOMETER

TRANSMISSION

AUTOMATIC DISPLAY

TABLET TERMINAL

SOFTWARE SUPPORT
(INFORMATION FEEDBACK)

ANALYZE CALORIE EXPENDITURE,
SLEEP TIME,
MENTAL STRESS, AND THE LIKE

PHYSICIAN IN CHARGE OF
USER, FAMILY, OR THE LIKE

ANALYSIS CENTER

SUPPORT (E.G., FOOD AND HEALTHY RELATED PRODUCTS)

ORGANIZATION THAT SUPPORTS
USER WITH RESPECT TO MEDICAL
CARE, FOOD AND SHELTER

EP 2 893 878 A1

FIG. 3

## FIG. 4A

## FIG. 4B

FIG. 5

# FIG. 6

Health Watch A6

2333 kcal/day

EXIT

10:59:36 MAY 18, 2012

A1

A11

11/08/20 土
IT52:13

IMPORT

A12

SET PERSONAL INFOR-MATION

SET CLOCK

DELETE

A2 {

EXERCISE MEASUREMENT        A21

c:¥MYDATA¥20120516232342.csv

IMPORTED DATA

c:¥MYDATA¥20120516232342.csv
c:¥MYDATA¥20120516174505.csv
c:¥MYDATA¥20120514234358.csv
c:¥MYDATA¥20120513082912.csv
c:¥MYDATA¥20120511165904.csv
c:¥MYDATA¥20120511104543.csv
c:¥MYDATA¥20120510182456.csv
c:¥MYDATA¥20120510081737.csv
c:¥MYDATA¥20120509232002.csv
c:¥MYDATA¥20120507235009.csv
c:¥MYDATA¥20120506231129.csv
c:¥MYDATA¥20120430110851.csv
c:¥MYDATA¥20120426235503.csv

A22

A31   A3   A32

HR TREND

ANALYZE

SAVE

DELETE

A4

SET COEFFI-CIENT

SET HR$_0$

A5

A51       A52

EP 2 893 878 A1

# FIG. 7

BASAL HEART RATE HR$_0$ [ 50 ]    [ BACK ]

[ BODY MOTION COEFFICIENT X | 5.00 | CALCULATE x ]

B1

BODY MOTION CORRECTION VALUE $\alpha$ [ 1.20 ]

RESTING COEFFICIENT Y [ 1.50 ]

RESTING CORRECTION VALUE $\beta$ [ 1.00 ]

STRESS COEFFICIENT [ 1.60 ]

SLEEP COEFFICIENT [ 1.11 ]

CALORIE EXPENDITURE FOR CORRECTION (Mets) [ 3.00 ]  B2

ACCELERATION COEFFICIENT [ 5 ]

FIG. 8

CALORIE EXPENDITURE 228.172kcal/24.12h

# FIG. 9A

Mental Stress[h] (vertical axis): 0.5, 1.0, 1.5, 2.0, 2.5

D1
D2
D3
D4

MENTAL STRESS TIME ZONE

EVALUATION OF DEEP SLEEP TIME

ACT(−) TIME ZONE

PHYSICAL STRESS TIME ZONE (Physical S)

ACT(+) TIME ZONE

Physical Stress[h] (horizontal axis): 0.5, 1.0, 1.5, 2.0, 2.5

# FIG. 9B

Health Watch
(CONVERTED TO 24-HOUR VALUE)

DEEP SLEEP TIME

ACT(+)

2270
kcal/day

ACT(−)

Physical S    Mental S

EP 2 893 878 A1

# FIG. 10

C:¥MYDATA¥20120726234419.osv  [READ]

START [2012/07/26 23:44:19]  END [2012/07/27 16:10:31]  MEASUREMENT DURATION (h) [16:44]

[BACK]

ID [10001001]

AGE [70]  SEX 1:MALE 0:FEMALE [1]  HEIGHT (cm) [169]  WEIGHT (kg) [61]

[HR TREND]

BASAL HEART RATE $HR_0$ [50]

BODY MOTION COEFFICIENT X [5.00]  RESTING COEFFICIENT y [1.50]  STRESS COEFFICIENT [1.60]  SLEEP COEFFICIENT [1.11]

[CALCULATE]

BODY MOTION CORRECTION VALUE $\alpha$ [1.20]  RESTING CORRECTION VALUE $\beta$ [1.00]  ACCELERATION COEFFICIENT [10]

## BASIC CALCULATION

BODY SURFACE AREA  RSA [1.7]

BASAL METABOLISM

MALE BMm [1319] [*]

FEMALE BMf [1190]

BODY MOTION TIME (h) [5.58]

RESTING TIME (h) [10.86]

CALORIE EXPENDITURE DURING BODY MOTION (kcal) [998.62]

CALORIE EXPENDITURE AT REST (kcal) [825.92]

PHYSICAL AVERAGE [1.28]

MENTAL AVERAGE [1.55]

PHYSICAL STRESS (h) [3.14]

MENTAL STRESS (h) [1.56]

DEEP SLEEP TIME (h) [5.18]

Health Watch (CONVERTED TO 24-HOUR VALUE)

ACT (+)  DEEP SLEEP TIME

Physical S

2664 kcal/day

Mental S  ACT (−)

[PRINT]

EP 2 893 878 A1

## INTERNATIONAL SEARCH REPORT

| International application No. |
| --- |
| PCT/JP2013/073809 |

**A. CLASSIFICATION OF SUBJECT MATTER**
*A61B5/22*(2006.01)i, *A61B5/11*(2006.01)i, *A61B5/0245*(2006.01)n

According to International Patent Classification (IPC) or to both national classification and IPC

**B. FIELDS SEARCHED**

Minimum documentation searched (classification system followed by classification symbols)
A61B5/22, A61B5/11, A61B5/0245

Documentation searched other than minimum documentation to the extent that such documents are included in the fields searched
Jitsuyo Shinan Koho        1922-1996   Jitsuyo Shinan Toroku Koho   1996-2013
Kokai Jitsuyo Shinan Koho   1971-2013   Toroku Jitsuyo Shinan Koho   1994-2013

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2004-223271 A  (Seiko Epson Corp.), 12 August 2004 (12.08.2004), entire text; all drawings (particularly, paragraphs [0007], [0008], [0059] to [0071]; fig. 10) & US 6030342 A          & US 6287262 B1 & EP 845241 A1          & EP 1424038 A1 & WO 1997/047239 A1 | 1-5,13, 15-17,19 |
| X | JP 8-112270 A  (Matsushita Electric Works, Ltd.), 07 May 1996 (07.05.1996), abstract; examples (Family: none) | 1,6-8,13, 15-17,19 |

| ☒ | Further documents are listed in the continuation of Box C. | | ☐ | See patent family annex. |
| --- | --- | --- | --- | --- |

\* Special categories of cited documents:
"A"  document defining the general state of the art which is not considered   to be of particular relevance
"E"  earlier application or patent but published on or after the international filing date
"L"  document which may throw doubts on priority claim(s) or which is cited to establish the publication date of another citation or other special reason (as specified)
"O"  document referring to an oral disclosure, use, exhibition or other means
"P"  document published prior to the international filing date but later than the priority date claimed

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

| Date of the actual completion of the international search 20 September, 2013 (20.09.13) | Date of mailing of the international search report 01 October, 2013 (01.10.13) |
| --- | --- |
| Name and mailing address of the ISA/ Japanese Patent Office | Authorized officer |
| Facsimile No. | Telephone No. |

Form PCT/ISA/210 (second sheet) (July 2009)

32

**INTERNATIONAL SEARCH REPORT**

International application No.

PCT/JP2013/073809

C (Continuation).   DOCUMENTS CONSIDERED TO BE RELEVANT

| Category* | Citation of document, with indication, where appropriate, of the relevant passages | Relevant to claim No. |
|---|---|---|
| X<br>A | JP 4-180730 A  (Atsufuku TAKARA),<br>26 June 1992 (26.06.1992),<br>entire text; all drawings<br>& US 5267568 A          & GB 2251490 A<br>& DE 4133608 A          & FR 2669450 A<br>& BE 1004497 A          & CH 685917 A<br>& SE 9103165 A          & AU 640044 B<br>& AU 8450591 A          & IT MI912536 A<br>& KR 10-0138506 B | 1,9,13,<br>15-17,19<br>10-12 |
| X | JP 2002-149831 A  (Sanyo Electric Co., Ltd.),<br>24 May 2002 (24.05.2002),<br>carrying-out mode 1; paragraph [0049] and<br>thereafter<br>& US 2002/0059081 A1 | 14-16,18,20 |
| X | JP 9-294727 A  (Seiko Epson Corp.),<br>18 November 1997 (18.11.1997),<br>entire text; all drawings<br>(Family: none) | 1-2 |
| A | JP 9-56705 A  (Matsushita Electric Works, Ltd.),<br>04 March 1997 (04.03.1997),<br>entire text; all drawings (particularly, fig.<br>2, 4)<br>(Family: none) | 1-5 |
| A | JP 2000-215 A  (Arata NEMOTO),<br>07 January 2000 (07.01.2000),<br>entire text; all drawings (particularly, fig.<br>2)<br>(Family: none) | 1,6-8 |
| A | JP 2009-148372 A  (Panasonic Electric Works<br>Co., Ltd.),<br>09 July 2009 (09.07.2009),<br>entire text; all drawings (particularly, fig.<br>2)<br>(Family: none) | 1,9-12 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009285498 A